# EUROPEAN PATENT SPECIFICATION

(11) **EP 4 192 333 B1**
(45) Date of publication and mention of the grant of the patent: **01.10.2025**
(21) Application number: 21759476.1
(22) Date of filing: 03.08.2021
(51) Int. Cl.: A61B 5/00, A61B 5/0205, A61B 5/16, A61B 5/024

(54) **STRESS DETERMINATION AND MANAGEMENT TECHNIQUES**
STRESSBESTIMMUNGS- UND -VERWALTUNGSTECHNIKEN
TECHNIQUES DE DÉTERMINATION ET DE GESTION DE STRESS

(30) Priority: 07.08.2020 US 202063062818 P
(43) Date of publication of application: 14.06.2023
(73) Proprietor: Fitbit LLC, San Francisco, CA 94105 (US)
(72) Inventor: ABDEL-GHAFFAR, Samy Ahmed, San Francisco, California 94105 (US); TRUAX, Stuart Blanché, San Francisco, California 94105 (US); HENEGHAN, Conor Joseph, San Francisco, California 94105 (US); PEREZ, Elena, San Francisco, California 94105 (US); THOMSEN, Julia Marie Dorothea, San Francisco, California 94105 (US)
(74) Representative: Maikowski & Ninnemann Patentanwälte Partnerschaft mbB
(86) International application number: PCT/US2021/044300
(87) International publication number: WO 2022/031671

(56) References cited:
- US-A1- 2017 319 122
- US-A1- 2018 107 943
- US-A1- 2018 220 947
- US-A1- 2018 310 867

## Description

### RELATED APPLICATIONS

This application claims the benefit of U.S. Provisional Patent Application No.: 63/062,818, filed on August 7, 2020.

### BACKGROUND

Recent advances in technology, including those available through consumer devices, have provided for corresponding advances in health detection and monitoring. For example, devices such as fitness trackers and smart watches are able to determine information relating to the pulse or motion of a person wearing the device. Due to capabilities of conventional devices, however, a technical problem exists relating to the amount and types of health information able to be determined using such devices, as collection of such information has been limited. In particular, conventional devices are limited in being able to detect parameters that accurately and automatically indicate stress of the user. Examples for conventional devices/techniques are described in US 2018/107943 A1, US 2017/319122 A1, and US 2018/310867 A1.

Thus, the present disclosure is directed to a technical solution/benefit to the aforementioned technical problem. Accordingly, the present disclosure is directed to a system and method for calculating a stress score for a user of a wearable device. In particular embodiments, for example, the stress score can be calculated using electro-dermal activity (EDA) data collected from an EDA sensor while the user is wearing the wearable device. More specifically, the sympathetic nervous system can trigger micro-perspiration throughout a person's body, so conductance between the EDA sensor on the wearable device and a hand or fingertip of the user will increase as the perspiration levels increase. As such, when a palm or finger of the user is placed adjacent to the EDA sensor, the EDA sensor can generate data that can be used by the wearable device to accurately and automatically calculate stress of the user.

### BRIEF DESCIPTION

Aspects and advantages of the invention will be set forth in part in the following description, or may be obvious from the description, or may be learned through practice of the invention.

The subject-matter of the claims is presented. The present disclosure is directed to a method for accurately and automatically calculating a stress score for a user of a wearable device. The method includes receiving, from one or more external sensors on the wearable device, first feature data corresponding to a state of the user of the wearable device. The method also includes obtaining, via a processor of the wearable device, second feature data corresponding to the state of the user, the second feature data provided by the user. Further, the method includes calculating, via the processor of the wearable device, a stress score using the first feature data and the second feature data. In addition, the method includes performing, via the processor of the wearable device, at least one action based at least in part upon the calculated stress score.

In an embodiment, the stress score represents at least one of a current stress level or a stress resilience level of the user of the wearable device.

The first feature data includes electro-dermal activity (EDA) data captured using at least one external EDA sensor on the wearable device. In such embodiments, the external EDA sensor(s) may be mounted on a side of the wearable device away from a wrist of the user.

The first feature data and the second feature data includes feature data selected from sleep features, activity features, and heart features. Calculating the stress score using the first feature data and the second feature data further comprises calculating the stress score as a weighted sum of the sleep features, the activity features, and the heart features.

In additional embodiments, the sleep features may include at least one of restlessness, fragmentation, sleep reservoir level, deep/REM sleep duration, deep sleep latency, or nightmare occurrence.

In certain embodiments, the activity features may include at least one of active zone minutes or activity level, exercise or activity metrics, exertion metrics, activity type, movement patterns, or step count or movement.

In further embodiments, the heart features may include at least one of deep sleep heart rate variability (HRV), elevated heart rate (HR) at rest, sleeping HR above resting heart rate (RHR).

In still additional embodiments, the first feature data and the second feature data may include feature data selected from at least one of: fitness fatigue score, blood pressure, blood composition, respiration rate, temperature, metabolic data, blood sugar level, body weight or composition, psychological state, perceived stress, depression, vocal prosody/tone/pressure, blood cortisol/epinephrine/norepinephrine levels, low-density lipoprotein (LDL) levels, BMI x exercise, gender-specific values, or mood log data.

In another embodiment, the action(s) may include at least one of generating an interface, providing a notification, modifying an operation of the wearable device, providing a recommendation for the user, or transmitting data for analysis.

In another aspect, the present disclosure is directed to a wearable computing device. The wearable device includes one or more sensors, at least one processor, and at least one memory device having instructions that, when executed by the at least one processor, cause the wearable computing device to receive, from the one or more sensors, first feature data corresponding to a state of a user of the wearable device, obtain second feature data corresponding to the state of the user, the second feature data provided by the user, calculate a stress score using the first feature data and the second feature data, and perform at least one action based at least in part upon the calculated stress score.

### BRIEF DESCRIPTION OF THE DRAWINGS

Various embodiments in accordance with the present disclosure will be described with reference to the drawings, in which:
FIGS. 1A and 1B illustrate an example device that can be used to obtain and analyze user health information in accordance with various embodiments.
FIG. 2 illustrates an example set of devices that are able to communicate in accordance with various embodiments.
FIG. 3 illustrates an example stress score algorithm that can be utilized in accordance with various embodiments.
FIG. 4 illustrates example interfaces that can be provided in accordance with various embodiments.
FIG. 5 illustrates example interfaces that can be provided in accordance with various embodiments.
FIG. 6 illustrates example interfaces that can be provided in accordance with various embodiments.
FIG. 7 illustrates an example process for determining a stress score that can be utilized in accordance with various embodiments.
FIG. 8 illustrates an example process for monitoring stress for a user that can be utilized in accordance with various embodiments.
FIG. 9 illustrates an example environment in which aspects of various embodiments can be implemented.

### DETAILED DESCRIPTION

In the following description, various embodiments will be described. For purposes of explanation, specific configurations and details are set forth in order to provide a thorough understanding of the embodiments. However, it will also be apparent to one skilled in the art that the embodiments may be practiced without the specific details. Furthermore, well-known features may be omitted or simplified in order not to obscure the embodiment being described.

As computing devices become more ubiquitous and portable, many advantages are being seen in the field of health monitoring and diagnostics. Computing devices, particularly ones that can be worn or carried by a user, may include one or more sensors to detect physiological information about the user and/or the environment around the user. This information can be used to observe, detect, or diagnose various health conditions outside of a traditional clinic or laboratory setting. For example, in the context of monitoring stress, portable or wearable electronic devices may be able to detect when the sympathetic nervous system of the user triggers micro-perspiration throughout the user's body. Additionally, a computing device may be able to record and interpret the detected information about the user and/or environment, to determine a health assessment. As in the previous example, a wearable electronic device may be able to record a relatively high frequency of a skin conductance response (SCR), which looks at the number of spikes in the skin conductance within a sliding window of time, and generate an assessment that the user is experiencing stress.

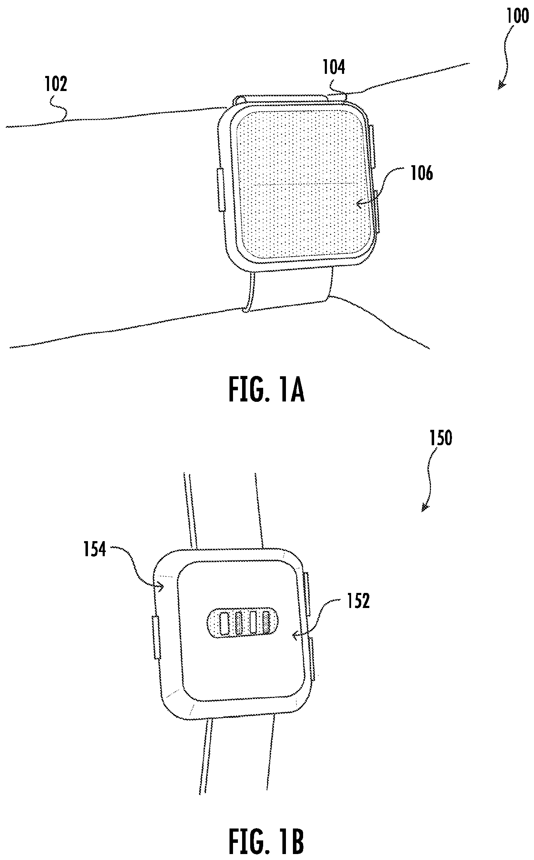

Approaches in accordance with various embodiments provide for determination, prediction, and/or monitoring of factors that can be indicative of stress or other such states of a user. In at least one embodiment, this determination can be made based, at least in part, upon data collected by a wearable computer, such as a device 100 illustrated in the embodiment of FIG. 1A. In various embodiments discussed herein, a person can wear or utilize the device 100 that is able to automatically measure or determine at least some aspects of the health or wellbeing of the person. In particular, as shown in FIG. 1A, the device 100 is a smart watch 104, although other devices such as smart or connected fitness bands or trackers, watches, rings, earbuds, phones, clothing, and the like can be utilized as well in the various embodiments. In this example, the person can wear the device 100 on an arm 102 or wrist, and can view content, as may include health information, on a display 106 of the device. **In** many embodiments, the display 106 is a touch sensitive display that allows the person to input or annotate information about that person's health or status as discussed elsewhere herein.

Referring now to FIG. 1B, the device 100 may include various measurement components 152, 154 (also referred to herein as sensors) as illustrated in the back view 150 of the device 100. More specifically, as shown, the device 100 may include one or more internal sensors 152 and/or one or more external sensors 154, which may include any suitable type of sensors, such as EDA sensors and/or motion and temperature sensors, that can be used to measure or detect information about the user. Further, in an embodiment, the measurement components 152, 154 can include, or relate to, an optical measurement sub-system. In this example, the optical measurement sub-system includes at least one optical emitter and at least one optical receiver. The emitter can emit light of one or more wavelengths that can be reflected from the surface of the wearer's skin, or diffusely reflected after traveling, under the surface, and detected by at least one of the emitters. Such an optical assembly can enable the smart watch to measure various types of information during times in which a person is wearing the device. In yet another embodiment, the external sensor(s) 154 may include an EDA sensor that is easily accessible by a palm or finger of the user, such that the user can easily make contact with the EDA sensor so that the sensor can generate data that can be used by the device 100 to accurately and automatically calculate stress of the user. Accordingly, the present disclosure is tied to the practical application of accurately and automatically calculating stress of the user through the EDA sensor(s) 154.

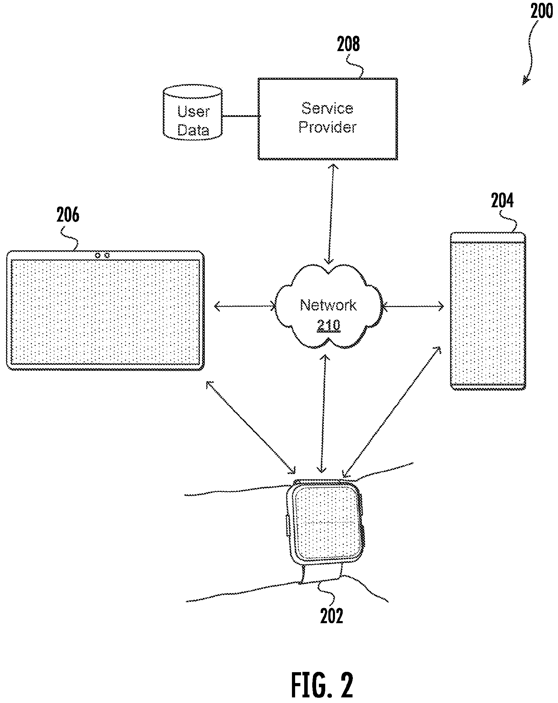

FIG. 2 illustrates an example environment 200 in which aspects of various embodiments can be implemented. In this example, a person might have a number of different devices that are able to communicate using at least one wireless communication protocol. In this example, the user might have a smart watch 202 or fitness tracker, which the user would like to be able to communicate with a smart phone 204 and a tablet computer 206. The ability to communicate with multiple devices can enable a user to obtain information from the smart watch 202, such as heart rate data captured using a sensor on the smart watch, using an application installed on either the smart phone 204 or the tablet computer 206. The user may also want the smart watch 202 to be able to communicate with a service provider 208, or other such entity, that is able to obtain and process data from the smartwatch and provide functionality that may not otherwise be available on the smartwatch or the applications installed on the individual devices. The smart watch 202 may be able to communicate with the service provider 208 through at least one network 210, such as the Internet or a cellular network, or may communicate over a wireless connection such as Bluetooth^{®} to one of the individual devices, which can then communicate over the at least one network. There may be a number of other types of, or reasons for, communications in various embodiments.

In addition to simply being able to communicate, a user may also want the devices to be able to communicate in a number of ways or with certain aspects. For example, the user may want communications between the devices to be secure, particularly where the data may include personal health data or other such communications. The device or application providers may also be required to secure this information in at least some situations. The user may want the devices to be able to communicate with each other concurrently, rather than sequentially. This may be particularly true where pairing may be required, as the user may prefer that each device be paired at most once, or that not manual pairing is required. The user may also desire the communications to be as standards-based as possible, not only so that little manual intervention is required on the part of the user but also so that the devices can communicate with as many other types of devices as possible, which is often not the case for various proprietary formats. A user may thus desire to be able to walk in a room with one device and have such device automatically communicate with another target device with little to no effort on the part of the user. In various conventional approaches, a device will utilize a communication technology such as Wi-Fi to communicate with other devices using wireless local area networking (WLAN). Smaller or lower capacity devices, such as many Internet of Things (IoT) devices, instead utilize a communication technology such as Bluetooth^{®}, and in particular Bluetooth Low Energy (BLE) that has very low power consumption.

In further embodiments, the environment 200 illustrated in FIG. 2 enables data to be captured, processed, and displayed in a number of different ways. For example, data may be captured using sensors on a smart watch 202, but due to limited resources on that smart watch the data may be transferred to a smart phone 204 or the service provider 208 (or a cloud resource) for processing, and results of that processing may then be presented back to that user on the smart watch 202, smart phone 204, or another such device associated with that user, such as the tablet computer 206. In at least some embodiments, a user may also be able to provide input such as health data using an interface on any of these devices, which can then be considered when making that determination.

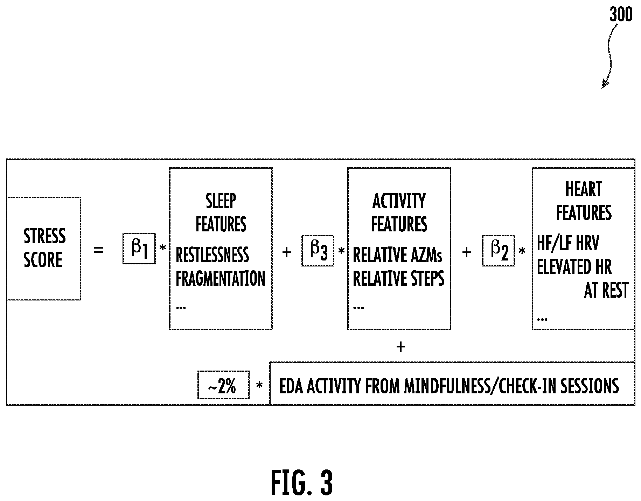

In at least one embodiment, data determined for a user can be used to determine state information, such as may relate to a current stress level or state of that user. At least some of this data can be determined using sensors or components able to measure or detect aspects of a user, while other data may be manually input by that user or otherwise obtained. In at least one embodiment, a stress determination algorithm can be utilized that takes as input a number of different inputs, where different inputs can be obtained manually, automatically, or otherwise. In at least one embodiment, such an algorithm can take various types of factors and use these to generate a stress score. One such stress score can be calculated as illustrated in approach 300 of FIG. 3. In this example, the stress score is calculated as a weighted sum of different types of factors. In at least one embodiment, these types of factors can include sleep features (e.g., restlessness, fragmentation), activity features (e.g., relative AZMs, relative steps), and/or heart features (e.g., HF/LF HRV, elevated heart rate at rest), as well as inputs such as electro-dermal activity (EDA), where EDA can be used to measure a galvanic skin response. In at least one embodiment, these factors can be normalized, and then weights determined through testing, machine learning, or other such approaches.

In at least one embodiment, these factors can be made up of at least twelve different metrics. It should be understood, however, that different numbers, selections, types, or variations can be used with different algorithms in accordance with various embodiments. A first feature type, relating to sleep features, can include features such as the following. A sleep score restlessness value can provide a measure of an amount of movement during sleep, which may be normalized for a person over a period such as 30 days. Restless sleep is a known physiological stress marker. A fragmentation feature can indicate the number of times a person was awake for more than a threshold amount of time, such as 30 minutes (WASO), which may be normalized as an average over a period such as 30 days. Fragmented sleep is also known to be a physiological stress marker. A sleep reservoir level indicates how well a person has slept over, for example, a last week with days further away affecting the score less, normalized over population level. This metric analyzes the user's sleep from the last week, to account for the fact that multiple bad nights of sleep cannot always be recovered from in a single night of sleep. Overall sleep duration also can cause poor emotion regulation, and so situations that may not induce stress after a good night's sleep can become stressful. In at least one embodiment, a set of constants can be determined and/or utilized to calculate a rate at which a user's sleep reservoir level depletes. A sleep reservoir level can represent an accumulated amount of restful sleep that a user has had over a recent period of time, such as a last seven days of sleeping. If the user has had a somewhat fitful sleep, very shallow sleep, or very short sleep, this reservoir level will deplete, getting closer to zero but at different rates. If the user is very well rested, getting at least seven hours of sleep each of the past seven days, then this value may be closer to a maximum normalized value of one (1). Such an approach can map sleep calculations into a normalized sleep reservoir scale, using specific constants that can determine a rate at which a user depletes his or her sleep, and how a particular sleeping period may accumulate in terms of sleep. In one embodiment, a first period of sleep is not counted as restful sleep, with only sleep beyond that initial period being determined to count as restful sleep. After this initial period the user can be determined to be accumulating sleep. A state or type of sleep after that initial period can then determine an amount or rate of restfulness that is being restored.

A deep and/or REM sleep duration can indicate, for example, minutes of deep sleep and percentage of REM (rapid eye movement) sleep for a most recent night, normalized using demographic group average over a period such as the last 30 days. A deep sleep latency feature can indicate a number of minutes before first entering deep sleep after falling asleep, which can be normalized using a personal average over, for example, the last 30 days. Longer latency between the start of sleep and the first deep sleep period is known to be a physiological stress marker.

In at least one embodiment, there can be a number of different activity features. This can include an active zone minutes (AZMs) feature that includes a weekly number of active zone minutes, normalized using a population level relative to, for example, a recommended 150/week. Feature value can increase when AZMs are higher or lower than a range around 150. **In** at least some embodiments, these minutes can be determined using sensors, such as motion and heart rate sensors, on a device. Too little weekly activity can result in increased cortisol levels and make one more susceptible to perceiving events as stressors, since mild to moderate exercise acts as a "shield" against stress. Too much weekly activity can result in fatigue, as exercise is by definition a stressor. A stressor represents a stimulus or event that cases a state of strain or tension, and thus results in a stress response. A stress response, or symptom, represents the physiological and psychological changes that a body undergoes in response to a stressor in an attempt to maintain homeostasis. Multiple stress responses can be elicited by a single stressor. A psychological stress response represents a relationship between a person and the environment that is appraised by the person as taxing or exceeding his or her resources and endangering his or her well-being. Another feature can be a step count value, corresponding to a total number of steps over a period, such as per day. This can be normalized using demographic group average over a period such as 30 days. Too few daily steps can result in increased cortisol levels and make one more susceptible to perceiving events as stressors, since mild to moderate exercise acts as a "shield" against stress. Too many daily steps can result in fatigue, as exercise is by definition a stressor. There can be other factors indicative of activity as well, where different types of data are analyzed to determine different types, or amounts, of activity.

In at least one embodiment, there can be various heart features included in a stress determination. One heart feature is a deep sleep high-frequency (HF) over low-frequency (LF) heart rate value (HRV). In at least one embodiment, this captures sympathovagal balance between sympathetic/parasympathetic activities, and can be normalized by demographic group average over a period such as 30 days. When insufficient deep and REM sleep occur, emotional regulation becomes more difficult, and so situations that may not induce stress after a good night's sleep can become stressful. Heart rate variability (HRV) quantifies the variability in time between heart-beats. Many different HRV metrics exist, and Low Frequency (LF) over High Frequency (HF) power quantifies symapthovagal balance, where a larger number means more SNS activity, and a lower number means more PNS activity. This metric only considers LF/HR HRV during periods of deep sleep, when SNS is highest during the day, as it is the most restful sleep. Another feature can relate to elevated heart rate (HR) at rest, which can correspond to the amount and magnitude of resting HR that is above a threshold, as may be normalized using personal average over a period such as 30 days. Elevated heart rate while resting during the day is a sign of increased sympathovagal balance, which means too much sympathetic nervous system (SNS) activity and not enough parasympathetic nervous system (PNS) activity. While stress results in an increased sympathovagal balance, many other things can cause increased heart rate including anemia, caffeine, alcohol, fever, high or low blood pressure, electrolyte imbalance (possibly from dehydration), hyperthyroidism, smoking, or medications.

A sleeping heart rate above resting heart rate (RHR) can indicate a percentage of time during sleep that the user's HR is above resting HR, as may be normalized using an absolute metric as a raw percentage. Similar to Elevated HR at Rest, this is also a measure of sympathovagal balance, but quantifies HR during sleep instead of during the day. While many factors can cause this metric to be high, specifically drinking alcohol before sleep drives this to be high. A fitness fatigue score can be calculated, which balances the fatigue effects of exercise with fitness effects of exercise into a single score, as may be normalized using a personal min/max range over a period such as 30-90 days. This score measures the dual contributions of exercise to one's fitness level in the long term, and fatigue in the short term, as measured through heart rate. Similar to weekly activity and daily steps, high fatigue levels are by definition stress that require rest. Fitness fatigue can treat the human heart as a linear system. By measuring heart rate over a period of time, an accumulated representation of an amount of fatigue on a human body can be obtained, as quantified by a fitness fatigue score. When a user goes through a high heart rate event like an exercise, there will be a time when this score will go down, reflecting the fatigue effects of this exercise. After that period there will be a period where the score will go up, to reflect the fact that the body has become more fit and more resilient to exercise, and is better prepared for exercise events in the future.

In at least one embodiment, EDA data can also be considered as a stress determination factor as discussed above. Approaches for capturing or determining EDA data are described in co-pending application entitled DETECTION AND RESPONSE TO AROUSAL ACTIVATIONS, which is incorporated herein in its entirety for all purposes. Sympathetic nervous system activity causes perspiration, and EDA measures the amount of conductance across the user's skin to quantify current amounts of SNS activity. This metric does not measure PNS in at least some embodiments, and so it may not provide a true measure of sympathovagal balance, rather just SNS activity, unlike other heart-related metrics. A feature value can relate to EDA activity during meditation and check-in sessions. For example, if a user has logged meditation or "check-in" sessions using the EDA session, then their average EDA score across all sessions can be compared with their baseline value and the feature can receive a score of, for example, 0, 1, or 2 points. If no sessions were logged, they may receive 1 point. In one embodiment, users who did not log EDA that day, may get 1 point, while users who did a guided EDA session get 2 points (regardless of alignment), and users who did an unguided EDA session, use an average of the session relative to the last 30 days of valid EDA sessions. If session is higher than average, those users get 0 points. If the session is around average, the users get 1 point. If session is less than the average, the users get 2 points.

In at least one embodiment, a weighting of the EDA in a stress score calculation can vary, as may depend in part upon factors such as an accuracy of the EDA values or a relative importance of that data for an individual user. In at least one embodiment, EDA can serve as a proxy for quantifying the amount of sympathetic nervous system activity. This data can represent this activity happening momentarily. The sympathetic nervous system can trigger micro-perspiration throughout a person's body, so the conductance between a sensor and a hand or fingertip of a user will increase as the perspiration levels increase. When greater conductance is detected, this can be representative of higher sympathetic nervous system activity. In at least one embodiment, at least two different EDA metrics can be monitored. A first metric is a tonal metric called skin conductance level, which looks at the absolute level over time, and whether that value is increasing or decreasing. A second metric represents a skin conductance response (SCR), which looks at the number of spikes in the skin conductance within a sliding window of time, such as a window of one minute in duration. SCR thus can represent a count per minute of spikes in the EDA conductance level. In at least one embodiment, a continuous EDA determination can be made for such purposes, while a discrete or periodic EDA signal may not support such granular determinations.

In at least one embodiment, an algorithm can utilize at least some of these and/or other such features to generate a stress score that is representative of a current, past, or future stress state of a user. A system or service utilizing such a score can provide a real-time metric that can be correlated with physical and/or perceived stress. Such a system may provide for manual input by a user, such has may relate to the collection of physical or perceived stress-related data. In at least one embodiment, such a system can capture perceived stress scale (PSS) survey results measuring chronic stress on a broad distribution of users. Such a system can also generate a daily physiological stress and/or stress capacity metric. In at least one embodiment, an algorithm can calculate stress scores that represent an "expert guided" determinations of physiological stress that can be inverted to reflect stress capacity and/or resilience. In at least one embodiment, low stress capacity or resilience scores can indicate high levels of stress, while high resilience scores can indicate readiness for the cognitive/emotional/physical demands of the day.

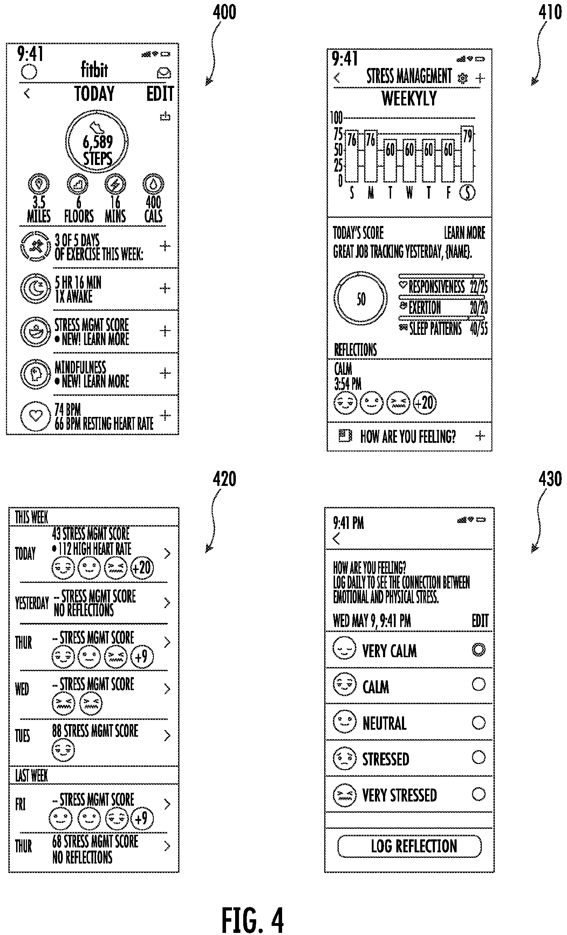

FIG. 4 illustrates example displays or interfaces that can be provided to a user in accordance with at least one embodiment. In this example, a first interface 400 provides information including a stress score field, as well as an option to obtain additional stress data. A second interface 410 provides a set of stress scores plotted over time, in this case over a certain week. Such information can help a user to identify trends, as well as to help to correlate events of different days with different stress levels or scores. As illustrated, a user may be able to provide feedback for different levels of stress, which can also be plotted by day for comparison. A third interface 420 can provide other information, such as mood, over that week or period of time. In at least one embodiment, there may be various sub-scores or components to a stress score, as may relate to responsiveness, exertion balance, or sleep patterns or scores. A fourth interface 430 can enable a user to provide feedback about their current mood or state.

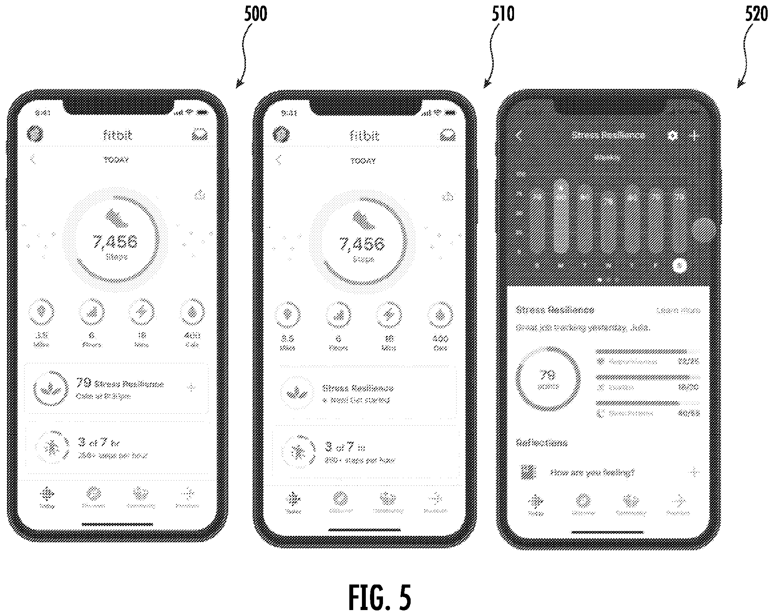

FIG. 5 illustrates additional interfaces that can be provided to a user in accordance with another embodiment. In this example, each interface presents information that is related to stress resilience, rather than a stress score that might be interpreted as an absolute level of stress by a user. A first interface 500 can present a stress resilience score, as well as a last presented stress state (and a time at which that state was presented). A second interface 510 can be presented to a user who has not yet started entering or obtaining stress resilience data, such as where at least some data or permission may be required from that user before presentation. A third interface 520 provides a different view of stress resilience over time, including data for individual stress resilience components as discussed previously. Such an interface can also allow a user to provide new, additional, or updated feedback that can be used in determining such values for presentation.

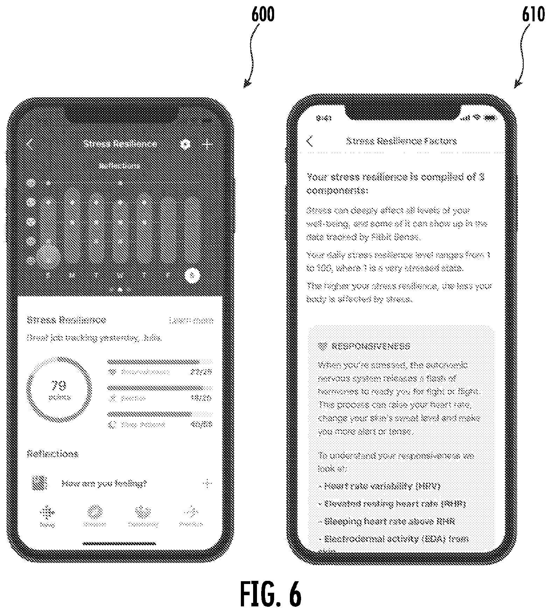

FIG. 6 illustrates additional interfaces that can be provided to a user in accordance with still another embodiment. A first interface page 600 presents another view of stress resilience data over time, particularly presenting distributions of stress levels or states for different days, rather than presenting resilience values. A second interface 610 presents information that helps a user to understand these scores, what goes into them, and what different scores represent. Various other data and interfaces can be used as well in accordance with various embodiments.

In at least one embodiment, data can be broken down into three stress score groups, namely an exertion group, a heart group, and a sleep pattern group. In such embodiments, the exertion group can include data for weekly activity, daily steps, and fitness fatigue score, or exertion balance. The heart group can include elevated HR at rest, sleeping HR above RHR, deep sleep HRV, and potentially EDA. This data can be representative of a user's nervous system, responsiveness, neural stimulation, neural gauge, and stimulation. The sleep pattern group can include data about sleep restlessness, sleep fragmentation, deep sleep latency, deep and REM sleep duration, and sleep reservoir level.

In at least one embodiment, a stress score can have values that range from 0 to 100, where a value of 100 can mean that a user's body has been showing signs of a lot of stress. Alternatively, a stress resilience score of 100 might mean that a user is showing little to no signs of stress. Other values or metrics can be used as well.

Such interfaces and information can attempt to provide a holistic management tool. Such an approach can thus analyze both physical stress and mental stress. An interface can be provided that quickly enables a user to obtain their daily or current stress score, along with other scores such as sleep scores and the like. Such information can also help a user to reflect on their mood and remind the user to log or provide that information. As mentioned, a user can also access additional data, such as a stress detail page where a user can obtain additional information about a current stress state or score, as well as the various components used for that determination. In at least some embodiments, a user may be able to drill into data components to obtain additional information of interest. In some embodiments there may be multiple views available as well, such as where a user wants to view trends over a day, week, or month, etc. As illustrated in various embodiments, sensors and devices may be able to provide data about physical stress, but a user may be the best source of mental stress data, at least for certain types of mental stress data. A user may be able to log a current mood or perceived stress level, or provide other such information. In at least one embodiment, a user can be prompted to enter mood or stress data after an EDA scan to provide for improved correlation. Such an approach can help to incorporate EDA data into a holistic view of a user's physical and mental health. In some embodiments, a device can provide for periodic or continuous EDA measurements, which can be used to dynamically update stress determinations over time.

In at least one embodiment, a stress score determination algorithm considers various factors that may impact a user's stress level. The weighting of these factors is determined across different types of users. In at least some embodiments these weightings can also be adapted for individual users to improve accuracy. For example, a sleep stages algorithm can be used to differentiate periods of sleep, which may have significant impact on stress levels for certain users. Further, looking at factors such as HRV in specific sleep states versus an entire night can provide additional insight. For example, combined deep and REM sleep duration can provide valuable insight as these periods are generally more restorative. There can be a significant difference between getting eight hours of light sleep versus four hours of light sleep and four hours of deep sleep and REM sleep. Further, mental stress factors may impact different users more heavily than physical stress factors, both of which can be considered by such an algorithm. Additional benefit can be obtained by looking at different types of metrics across different domains of exercise or exertion, sleep, and proxies for autonomic nervous system activity both with heart rate/heart rate variability as well as EDA or other such data. A useful benefit for a user can also be comparing physical to perceived stress levels, so a user can better understand their stress.

**In** at least some embodiments, a stress determination can be a linear or non-linear combination of weighted and normalized factors, where those factors may be normalized based on personal, demographic, or other such groupings. In some embodiments, some factors may need to be inverted where some represent higher stress levels while others represent lower stress levels. For example, in one embodiment inverted factors include deep and REM sleep duration, sleep reservoir level, and fitness fatigue score. Some factors can utilize a z-score-based approach, where a z-score of 1 represents one standard deviation above the mean, etc. As mentioned, factors can also be normalized over different periods of time and different groupings, such as per user or per demographic group. In some embodiments, a ranging function can be applied after z-scoring to put all values into a determined range, such as between 0 and 1. In some embodiments this can involve a linear rectification and interpolation.

In at least one embodiment, stress scores can be determined such that a single score, such as 80 on a scale of 100, represents the same stress level, or stress resilience, for all users. In other embodiments, values may mean different things to different users, such as where a first user might have an average stress level of 50 while another user might have an average stress level of 80, such that a value of 70 might mean different things to those two users.

Further, different factors might be considered in different algorithms or for different users. For example, a user who is very active might have a different algorithm utilized than a user who is not active, while different algorithms may also be utilized for different devices where different types of input are available, or where a user may have an option of deactivating certain sensors or restricting types of data from being collected. In one embodiment, at least three categories of factors can be collected, including sleep feature, activity features, and heart features (or "responsiveness" features). In some embodiments, EDA or similar data may be utilized as well. In some embodiments, factors within these categories that may be utilized can include restlessness, fragmentation, sleep reservoir level, deep/REM sleep duration, deep sleep latency, active zone minutes or activity level, step count or movement, deep sleep HRV, elevated HR at rest, sleeping HR above RHR, and fitness fatigue score. Other factors can include various exercise or activity metrics, as well as exertion balance metrics. Additional factors, if available, can include blood pressure, blood composition, respiration rate, temperature, metabolic data, blood sugar levels, body weight or composition, psychological state, perceived stress, depression, activity type, or current movement patterns (e.g., gait). Other factors may include nightmares, vocal prosody/tone/pressure, blood cortisol/epinephrine/norepinephrine levels, low-density lipoprotein (LDL) levels, BMI x exercise, gender-specific values, or mood log data.

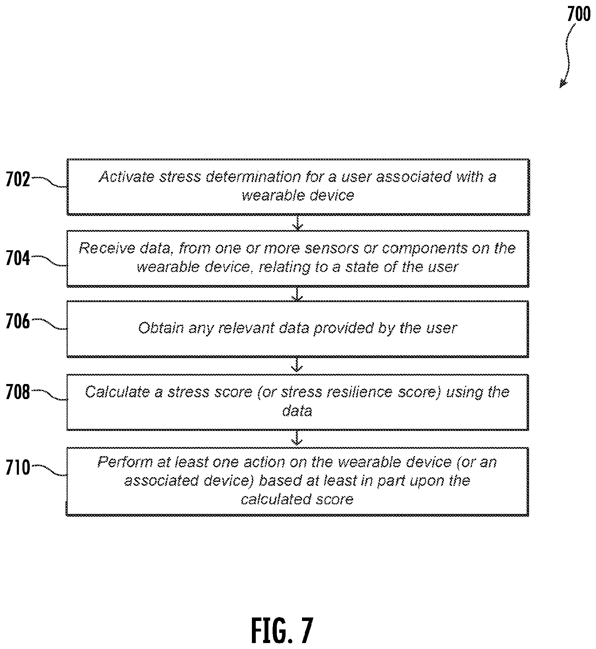

Referring now to FIG. 7, a flow diagram of one embodiment of an example process 700 for determining a stress score for a user that can be utilized is illustrated. It should be understood that processes discussed herein, there can be additional, fewer, or alternative steps performed in similar or alternative orders, or at least partially in parallel unless otherwise specifically stated. **In** this embodiment, as shown at (702), the process 700 includes activating stress determination for a user associated with a wearable device. This activation can come from a user, the wearable device, or an associated device, among other such sources. As part of the determination, as shown at (704), the process 700 includes receiving data, from one or more sensors or components on the wearable device, that relate to a state of the user, such as may relate to heart rate, sleep state, EDA, activity, or other such information discussed and suggested herein. As shown at (706), the process 700 includes obtaining additional data provided by the user, such as may relate to perceived state data of the user. Using at least some of this and other relevant data, as shown at (708), the process 700 includes calculating a stress score (or stress resilience score) for that user. This stress score or value can relate to various stress metrics, such as a current stress level or stress resilience level of the user. In this example, as shown at (710), the process 700 includes performing at least one action on the wearable device (or an associated device) based at least in part upon this calculated score. These actions can include any of a variety of different actions, as may relate to generating an interface, presenting data, providing a notification, updating, or modifying an operation of the wearable device, or performing another such action.

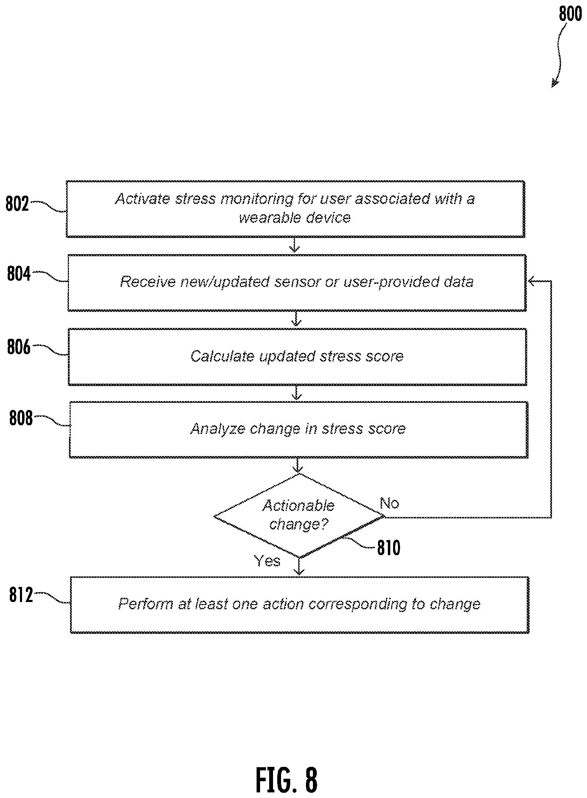

FIG. 8 illustrates another example process 800 for monitoring stress for a user that can be utilized in accordance with various embodiments. In this example, as shown at (802), the process 800 includes activating stress monitoring for a user associated with a wearable device. An initial stress determination can be performed, such as described with respect to FIG. 7. During the monitoring, as shown at (804), the process 800 includes receiving new or updated sensor data and/or user-provided data. As shown at (806), the process 800 includes calculating an updated stress score based at least in part upon this new or updated data. As shown at (808), the process 800 includes analyzing change in stress score, such as with respect to an immediately prior value determination. As shown at (810), the process 800 includes determining whether the change is an actionable change, such as a change that exceeds a change threshold, falls outside an acceptable range, or satisfies an action threshold. If not, then the process 800 can continue with new and updated data being received. If the change is determined to be an actionable change, then at least one action can be performed, as shown at (812) that corresponds to the change, where those actions can include those discussed with respect to the initial stress score calculation above.

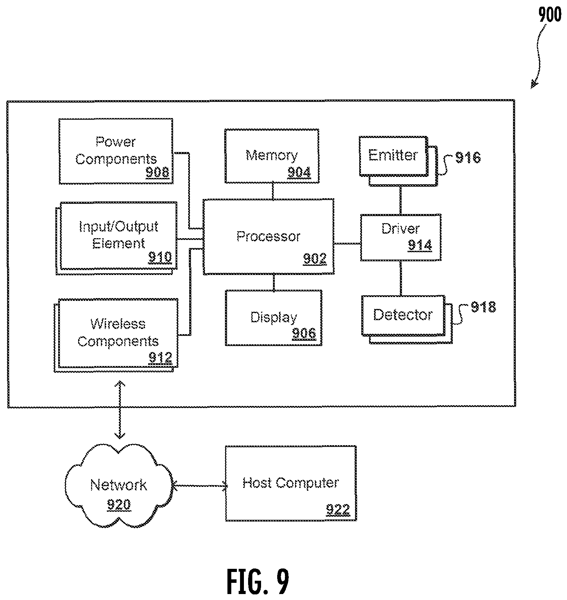

FIG. 9 illustrates components of an example system 900 that can be utilized in accordance with various embodiments. In this example, the system 900 includes at least one processor 902, such as a central processing unit (CPU) or graphics processing unit (GPU) for executing instructions that can be stored in a memory device 904, such as may include flash memory or DRAM, among other such options. As would be apparent to one of ordinary skill in the art, the device can include many types of memory, data storage, or computer-readable media, such as data storage for program instructions for execution by a processor. The same or separate storage can be used for images or data, a removable memory can be available for sharing information with other devices, and any number of communication approaches can be available for sharing with other devices. In addition, as shown, the system 900 includes any suitable display 906, such as a touch screen, organic light emitting diode (OLED), or liquid crystal display (LCD), although devices might convey information via other means, such as through audio speakers or projectors.

A tracker or similar device includes at least one motion detection sensor, which as illustrated can include at least one input/output (I/O) element 910 of the device. Such a sensor can determine and/or detect orientation and/or movement of the system 900. Such an element can include, for example, an accelerometer, inertial sensor, altimeter, or gyroscope operable to detect movement (e.g., rotational movement, angular displacement, tilt, position, orientation, motion along a non-linear path, etc.) of the device. An orientation determining element can also include an electronic or digital compass, which can indicate a direction (e.g., north or south) in which the device is determined to be pointing (e.g., with respect to a primary axis or other such aspect). The I/O element 910 may also be used for determining a location of the device (or the user of the device). Such a positioning element can include a GPS or similar location-determining element(s) operable to determine relative coordinates for a position of the device. Positioning elements may include wireless access points, base stations, etc., that may either broadcast location information or enable triangulation of signals to determine the location of the device. Other positioning elements may include QR codes, barcodes, RFID tags, NFC tags, etc., that enable the device to detect and receive location information or identifiers that enable the device to obtain the location information (e.g., by mapping the identifiers to a corresponding location). Various embodiments can include one or more such elements in any appropriate combination. The I/O elements 910 may also include one or more biometric sensors, optical sensors, barometric sensors (e.g., altimeter, etc.), and the like.

As mentioned above, some embodiments use the element(s) to track the location and/or motion of a user. Upon determining an initial position of a device (e.g., using GPS), the device of some embodiments may keep track of the location of the device by using the element(s), or in some instances, by using the orientation determining element(s) as mentioned above, or a combination thereof. As should be understood, the algorithms or mechanisms used for determining a position and/or orientation can depend at least in part upon the selection of elements available to the device. The example device also includes one or more wireless components 912 operable to communicate with one or more electronic devices within a communication range of the particular wireless channel. The wireless channel can be any appropriate channel used to enable devices to communicate wirelessly, such as Bluetooth, cellular, NFC, or Wi-Fi channels. It should be understood that the system 900 can have one or more conventional wired communications connections as known in the art. The system 900 also includes one or more power components 908, such as may include a battery operable to be recharged through conventional plug-in approaches, or through other approaches such as capacitive charging through proximity with a power mat or other such device. In some embodiments, the system 900 can include at least one additional I/O device 910 able to receive conventional input from a user. This conventional input can include, for example, a push button, touch pad, touch screen, wheel, joystick, keyboard, mouse, keypad, or any other such device or element whereby a user can input a command to the device. These I/O devices could even be connected by a wireless infrared or Bluetooth or other link as well in some embodiments. Some devices also can include a microphone or other audio capture element that accepts voice or other audio commands. For example, a device might not include any buttons at all, but might be controlled only through a combination of visual and audio commands, such that a user can control the device without having to be in contact with the device.

As mentioned, many embodiments include at least some combination of one or more emitters 916 and one or more detectors 918 for measuring data for one or more metrics of a human body, such as for a person wearing the tracker device. In some embodiments, this may involve at least one imaging element, such as one or more cameras that are able to capture images of the surrounding environment and that are able to image a user, people, or objects in the vicinity of the device. The image capture element can include any appropriate technology, such as a CCD image capture element having a sufficient resolution, focal range, and viewable area to capture an image of the user when the user is operating the device. Methods for capturing images using a camera element with a computing device are well known in the art and will not be discussed herein in detail. It should be understood that image capture can be performed using a single image, multiple images, periodic imaging, continuous image capturing, image streaming, etc. Further, a device can include the ability to start and/or stop image capture, such as when receiving a command from a user, application, or other device.

The emitters 916 and detectors 918 of FIG. 9 may also be capable of being used, in one example, for obtaining optical photoplethsymogram (PPG) measurements. Some PPG technologies rely on detecting light at a single spatial location, or adding signals taken from two or more spatial locations. Both of these approaches result in a single spatial measurement from which the heart rate (HR) estimate (or other physiological metrics) can be determined. In some embodiments, a PPG device employs a single light source coupled to a single detector (i.e., a single light path). Alternatively, a PPG device may employ multiple light sources coupled to a single detector or multiple detectors (i.e., two or more light paths). In other embodiments, a PPG device employs multiple detectors coupled to a single light source or multiple light sources (i.e., two or more light paths). In some cases, the light source(s) may be configured to emit one or more of green, red, and/or infrared light. For example, a PPG device may employ a single light source and two or more light detectors each configured to detect a specific wavelength or wavelength range. In some cases, each detector is configured to detect a different wavelength or wavelength range from one another. In other cases, two or more detectors configured to detect the same wavelength or wavelength range. In yet another case, one or more detectors configured to detect a specific wavelength or wavelength range different from one or more other detectors). In embodiments employing multiple light paths, the PPG device may determine an average of the signals resulting from the multiple light paths before determining an HR estimate or other physiological metrics. Such a PPG device may not be able to resolve individual light paths or separately utilize the individual signals resulting from the multiple light paths.

Referring still to FIG. 9, the system 900 may further include one or more processors 902 coupled to memory device 904, display 906, bus, one or more input/output (I/O) elements 910, and wireless networking components 912, among other such options. However, in certain embodiments, a display and/or I/O devices may be omitted. In an embodiment, the system 900 may be part of a wristband and the display 906 is configured such that the display faces away from the outside of a user's wrist when the user wears the wristband. In other embodiments, the display may be omitted and data detected by the system 900 may be transmitted using the wireless networking interface via near-field communication (NFC), Bluetooth, Wi-Fi, or other suitable wireless communication protocols over at least one network 920 to a host computer 922 for analysis, display, reporting, or other such use.

The memory 904 may include RAM, ROM, FLASH memory, or other non-transitory digital data storage, and may include a control program comprising sequences of instructions which, when loaded from the memory and executed using the processor 902, cause the processor 902 to perform the functions that are described herein. The emitters 916 and detectors 918 may be coupled to a bus directly or indirectly using driver circuitry by which the processor 902 may drive the emitters 916 and obtain signals from the detectors 918. The host computer 922 can communicate with the wireless networking components 912 via the one or more networks 920, which may include one or more local area networks, wide area networks, and/or internetworks using any of terrestrial or satellite links. In some embodiments, the host computer 922 executes control programs and/or application programs that are configured to perform some of the functions described herein.

In some embodiments, each emitter 916 can be individually controlled, or each detector 918 can be individually read out when multiple detectors are used, and in such embodiments, PPG sensor data along several different light paths can be collected. The control program can utilize the collected data to provide a more accurate estimation or HR and/or other physiological metrics. In related aspects, the processor 902 and other component(s) of the PPG device may be implemented as a System-on-Chip (SoC) that may include one or more central processing unit (CPU) cores that use one or more reduced instruction set computing (RISC) instruction sets, and/or other software and hardware to support the PPG device.

In various embodiments, the emitters 916 (or light sources) may include electronic semiconductor light sources, such as LEDs, or produce light using any of filaments, phosphors, or laser. In some implementations, each of the light sources emits light having the same center wavelength or within the same wavelength range. In other cases, at least one light source may emit light having a center wavelength that is different from another one of the light sources. The center wavelengths of the light emitted by the light sources may be in the range of 495 nm to 570 nm. For example, a particular green light source may emit light with a center wavelength of 528 nm. In other embodiments, one or more of the light sources may emit red light (e.g., 660 nm center wavelength) or IR light (e.g., 940 nm center wavelength). In some embodiments, one or more of the light sources may emit light with peak wavelengths typically in the range of 650 nm to 940 nm. For example, in various embodiments, a particular red light source may emit light with a peak wavelength of 660 nm, and one or more infrared light sources may emit light with peak wavelengths in the range of 750 nm to 1700 nm. By way of example and not limitation, a particular infrared light source may emit light with a peak wavelength of 730 nm, 760 nm, 850 nm, 870 nm, or 940 nm. In some cases, commercial light sources such as LEDs may provide output at about 20 nm intervals with a center wavelength tolerance of +/- 10 nm from the manufacturer's specified wavelength and thus one possible range of useful peak wavelengths for the light sources is 650 nm to 950 nm. The green light sources may be configured to emit light with wavelengths in the range of 495 nm to 570 nm. For example, a particular green light source may emit light with a wavelength of 528 nm. The green light sources may be equally spaced from light detectors as the pairs of red and infrared light sources. For example, if the distance between light detectors and a center of a first red light source is 2 mm, the distance between light detectors and a green light source may also be 2 mm (e.g., equidistant). In some other cases, the distance between the light detectors and one or more light sources is not equidistant. Further, in some embodiments, one or more of the light sources may comprise a single LED package that emits multiple wavelengths, such as green, red, and infrared wavelengths, at the same or substantially the same (e.g., less than 1 mm difference) location with respect to multiple detectors. Such LEDs may include multiple semiconductor elements co-located using a single die in a single package.

The spacing of the light sources may be measured from the side of the light source or the center of the light source. For example, the light sources may be configured such that the center of each light source is at a first distance from the edge of the closest one of the light detectors. In some embodiments, the first distance may be 2 mm. In some implementations, each light source is located at a second distance from the closest one of the light sources, and each light detector is located at a third distance from the closest one of the light detectors. In some embodiments, the second and third distances are identical to the first distance. In other embodiments, each of the second and third distances is different from the first distance. The second distance may be identical to or different from the third distance. The particular magnitude of the spacing may depend on a number of factors and this disclosure does not limit the embodiments to any particular spacing. For example, spacing in a range of 1 mm (or less) to 10 mm would be workable in various embodiments.

In some embodiments, independent control of all light sources is provided. In other embodiments, several light sources are controlled together as a gang or bank. A benefit of independent control of each light source, or independent readout from each of multiple detectors (e.g., obtaining independent signals based on the same or different light wavelengths from each of multiple detectors), is that a multiple light path approach may be used to improve the estimation of HR and/or other physiological metrics, as discussed further herein.

Light detectors may include one or more sensors that are adapted to detect wavelengths of light emitted from the light sources. A particular light source combined with a particular detector may comprise a sensor such as a PPG sensor. A first PPG sensor and a second PPG sensor can share components, such as the same light sources and/or detectors, or have different components and thus the term "PPG sensor," in addition to having its ordinary meaning, may refer to any of such arrangements although actual embodiments may use multiple components in implementing a PPG sensor. The term "PPG device," in addition to having its ordinary meaning, may refer to a device including a PPG sensor. A light detector, in an embodiment, may comprise one or more detectors for detecting each different wavelength of light that is used by the light sources. For example, a first detector may be configured to detect light with a wavelength of 560 nm, a second detector may be configured to detect light with a wavelength of 940 nm, and a third detector may be configured to detect light with a wavelength of 528 nm. Examples include photodiodes fabricated from semiconductor materials and having optical filters that admit only light of a particular wavelength or range of wavelengths. The light detectors may comprise any of a photodiode, phototransistor, charge-coupled device (CCD), thermopile detector, microbolometer, or complementary metal-oxide-semiconductor (CMOS) sensor. The light detectors may comprise multiple detector elements, as further described herein. One or more of the detectors may comprise a bandpass filter circuit.

In other embodiments, a detector may comprise one or more detectors configured to detect multiple wavelengths of light. For example, a single detector may be configured to tune to different frequencies based on data received from an electrical digital microprocessor coupled to detectors. In another way, the single detector may include multiple active areas where each active area is sensitive to a given range of wavelengths. In an embodiment, a single detector is configured to detect light with wavelengths in the red and IR frequencies and a second detector is configured to detect light with wavelengths in the green frequencies. Further, each of the light sources may use any of one or more different wavelengths of light as previously described.

In an embodiment, light detectors can be mounted in a housing with one or more filters that are configured to filter out wavelengths of light other than wavelengths emitted by light sources. For example, a portion of the housing may be covered with a filter which removes ambient light other than light in wavelengths emitted by light sources. For example, signals from light sources may be received at the light detectors through an ambient light filter that filters out an ambient light source that generates an ambient light with a wavelength that is different from the wavelength that is detected by the detector. Although LEDs and photodiodes are used as examples of the light sources and the light detectors, respectively, the techniques described herein may be extended to other types of light sources. For example, edge emitting lasers, surface emitting lasers, LED-pumped phosphors that generate broadband light. The techniques described herein may be extended to other combinations of light sources and detectors. For example, the PPG device may include (i) single or multiple LEDs and a multielement photodetector (e.g., a camera sensor), (ii) an LED array and single or multiple photodiodes, (iii) a broadband LED-pumped phosphor and detector array with wavelength selective filters on each detector, (iv) spatial light modulator (SLM) (e.g., a digital micromirror device [DMD] or a liquid crystal on silicon [LCoS] device) and single or multiple LEDs, other combinations thereof, or other configurations of light sources and detectors.

Certain flow diagrams are presented herein to illustrate various methods that may be performed by example embodiments. The flow diagrams illustrate example algorithms that may be programmed, using any suitable programming environment or language, to create machine code capable of execution by a CPU or microcontroller of the PPG device. In other words, the flow diagrams, together with the written description in this document, are disclosures of algorithms for aspects of the claimed subject matter, presented at the same level of detail that is normally used for communication of this subject matter among skilled persons in the art to which the disclosure pertains. Various embodiments may be coded using assembly, C, OBJECTIVE-C, C++, JAVA, or other human-readable languages and then compiled, assembled, or otherwise transformed into machine code that can be loaded into ROM, EPROM, or other recordable memory of the activity monitoring apparatus that is coupled to the CPU or microcontroller and then then executed by the CPU or microcontroller.

In an embodiment, signals obtained from multiple light paths may be processed to filter or reject signal components that are associated with motion of the user, using a computer program to identify the motion component of the signal and remove the identified motion component from the composite signal, leaving the cardiac component as a remainder or final signal.

In an embodiment, signals might be collected in variety of activities during day or at night, such as may relate to periods of walking, exercise, or sleep. Other on-device sensors including an accelerometer, gyroscope, or altimeter may be used to categorize or detect the activity, or human posture as a basis to develop the appropriate filters. These filters or signal processing methods might be used for targeted reduction of variability in the PPG data with multiple light paths. As an example, and not limitation, the accelerometer data can be used to develop signal processing methods to filter the data and look into a certain posture, removing other body orientations. This can help reduce the noise in the data and get a better assessment of the corresponding physiological variables for the corresponding light paths.

In various embodiments, approaches discussed herein may be performed by one or more of: firmware operating on a monitoring or tracker device or a secondary device, such as a mobile device paired to the monitoring device, a server, host computer, and the like. For example, the monitoring device may execute operations relating to generating signals that are uploaded or otherwise communicated to a server that performs operations for removing the motion components and creating a final estimate value for HR, SpO₂, and/or other physiological metrics. Alternatively, the monitoring device may execute operations relating to generating the monitoring signals and removing the motion components to produce a final estimate value for HR, SpO₂, and/or other physiological metrics local to the monitoring device. In this case, the final estimate may be uploaded or otherwise communicated to a server such as host computer that performs other operations using the value.

An example monitoring or tracker device can collect one or more types of physiological and/or environmental data from one or more sensor(s) and/or external devices and communicate or relay such information to other devices (e.g., host computer or another server), thus permitting the collected data to be viewed, for example, using a web browser or network-based application. For example, while being worn by the user, a tracker device may perform biometric monitoring via calculating and storing the user's step count using one or more sensor(s). The tracker device may transmit data representative of the user's step count to an account on a web service (e.g., www.fitbit.com), computer, mobile phone, and/or health station where the data may be stored, processed, and/or visualized by the user. The tracker device may measure or calculate other physiological metric(s) in addition to, or in place of, the user's step count. Such physiological metric(s) may include, but are not limited to: energy expenditure, e.g., calorie burn; floors climbed and/or descended; HR; heartbeat waveform; HR variability; HR recovery; respiration, SpO₂, blood volume, blood glucose, skin moisture and skin pigmentation level, location and/or heading (e.g., via a GPS, global navigation satellite system (GLONASS), or a similar system); elevation; ambulatory speed and/or distance traveled; swimming lap count; swimming stroke type and count detected; bicycle distance and/or speed; blood glucose; skin conduction; skin and/or body temperature; muscle state measured via electromyography; brain activity as measured by electroencephalography; weight; body fat; caloric intake; nutritional intake from food; medication intake; sleep periods (e.g., clock time, sleep phases, sleep quality and/or duration); pH levels; hydration levels; respiration rate; and/or other physiological metrics.

An example tracker or monitoring device may also measure or calculate metrics related to the environment around the user (e.g., with one or more environmental sensor(s)), such as, for example, barometric pressure, weather conditions (e.g., temperature, humidity, pollen count, air quality, rain/snow conditions, wind speed), light exposure (e.g., ambient light, ultra-violet (UV) light exposure, time and/or duration spent in darkness), noise exposure, radiation exposure, and/or magnetic field. Furthermore, a tracker device (and/or the host computer and/or another server) may collect data from one or more sensors of the device, and may calculate metrics derived from such data. For example, a tracker device may calculate the user's stress or relaxation levels based on a combination of HR variability, skin conduction, noise pollution, and/or sleep quality. In another example, a tracker device may determine the efficacy of a medical intervention, for example, medication, based on a combination of data relating to medication intake, sleep, and/or activity. In yet another example, a tracker device may determine the efficacy of an allergy medication based on a combination of data relating to pollen levels, medication intake, sleep and/or activity. These examples are provided for illustration only and are not intended to be limiting or exhaustive.

An example monitoring device may include a computer-readable storage media reader, a communications device (e.g., a modem, a network card (wireless or wired), an infrared communication device) and working memory as described above. The computer-readable storage media reader can be connected with, or configured to receive, a computer-readable storage medium representing remote, local, fixed and/or removable storage devices as well as storage media for temporarily and/or more permanently containing, storing, transmitting, and retrieving computer-readable information. A monitoring system and various devices also typically will include a number of software applications, modules, services, or other elements located within at least one working memory device, including an operating system and application programs such as a client application or Web browser. It should be appreciated that alternate embodiments may have numerous variations from that described above. For example, customized hardware might also be used and/or particular elements might be implemented in hardware, software (including portable software, such as applets) or both. Further, connection to other computing devices such as network input/output devices may be employed.

Storage media and other non-transitory computer readable media for containing code, or portions of code, can include any appropriate media known or used in the art, such as but not limited to volatile and non-volatile, removable and non-removable media implemented in any method or technology for storage of information such as computer readable instructions, data structures, program modules or other data, including RAM, ROM, EEPROM, flash memory or other memory technology, CD-ROM, digital versatile disk (DVD) or other optical storage, magnetic cassettes, magnetic tape, magnetic disk storage or other magnetic storage devices or any other medium which can be used to store the desired information and which can be accessed by a system device. Based on the disclosure and teachings provided herein, a person of ordinary skill in the art will appreciate other ways and/or methods to implement the various embodiments.

The specification and drawings are, accordingly, to be regarded in an illustrative rather than a restrictive sense. It will, however, be evident that various modifications and changes may be made thereunto without departing from the broader scope of the invention as set forth in the claims.

## Claims

1. A method for calculating a stress score for a user of a wearable device, the method comprising:
receiving (704), from one or more external sensors on the wearable device, first feature data corresponding to a state of the user of the wearable device, wherein the first feature data includes electro-dermal activity, EDA, data captured using at least one external EDA sensor on the wearable device;
obtaining (706), via a processor of the wearable device, second feature data corresponding to the state of the user, the second feature data provided by the user, and wherein the first feature data and the second feature data comprises feature data from sleep features, activity features, and heart features;
calculating (708), via the processor of the wearable device, a stress score using the first feature data and the second feature data, wherein the stress score is representative of a stress state of the user, and wherein calculating (708) the stress score using the first feature data and the second feature data further comprises calculating the stress score as a weighted sum of the feature data from the sleep features, the activity features, and the heart features;
and
performing (710), via the processor of the wearable device, at least one action based at least in part upon the calculated stress score, **characterized in that** a weighting of the feature data is determined for different types of users.

2. The method of claim 1, wherein the at least one external EDA sensor is mounted on a side of the wearable device away from a wrist of the user.

3. The method of claim 1, wherein the sleep features comprise at least one of restlessness, fragmentation, sleep reservoir level, deep/REM sleep duration, deep sleep latency, or nightmare occurrence.

4. The method of claim 1, wherein the activity features comprise at least one of active zone minutes or activity level, exercise or activity metrics, exertion metrics, activity type, movement patterns, or step count or movement.

5. The method of claim 1, wherein the heart features comprise at least one of deep sleep heart rate variability, HRV, elevated heart rate, HR, at rest, sleeping HR above resting

6. The method of any of the preceding claims, wherein the first feature data and the second feature data comprises feature data selected from at least one of: fitness fatigue score, blood pressure, blood composition, respiration rate, temperature, metabolic data, blood sugar level, body weight or composition, psychological state, perceived stress, depression, vocal prosody/tone/pressure, blood cortisol/epinephrine/norepinephrine levels, low-density lipoprotein, LDL, levels, BMI x exercise, gender-specific values, or mood log data.

7. The method any of the preceding claims, wherein the at least one action includes at least one of generating an interface, providing a notification, modifying an operation of the wearable device, providing a recommendation for the user, or transmitting data for analysis.

8. A wearable computing device, comprising:
one or more sensors;
at least one processor (902); and
at least one memory device (904) comprising instructions that, when executed by the at least one processor (902), cause the wearable computing device to:
receive (704), from the one or more sensors, first feature data corresponding to a state of a user of the wearable device, wherein the first feature data includes electro-dermal activity, EDA, data captured using an EDA sensor on the wearable device;
obtain (706) second feature data corresponding to the state of the user, the second feature data provided by the user, wherein the first feature data and the second feature data comprises feature data from sleep features, activity features, and heart features;
calculate (708) a stress score using the first feature data and the second feature data, wherein the stress score is representative of a stress state of the user, wherein the instructions further cause the wearable computing device to calculate the stress score as a weighted sum of the feature data from the sleep features, the activity features, and the heart features;
and
perform (710) at least one action based at least in part upon the calculated stress score, **characterized in that** a weighting of the feature data is determined for different types of users.

9. The wearable computing device of claim 8, wherein the sleep features comprise at least one of restlessness, fragmentation, sleep reservoir level, deep/REM sleep duration, deep sleep latency, or nightmare occurrence.

10. The wearable computing device of claim 8, wherein the activity features comprise at least one of active zone minutes or activity level, exercise or activity metrics, exertion metrics, activity type, movement patterns, or step count or movement.

11. The wearable computing device of claim 8, wherein the heart features comprise at least one of deep sleep heart rate variability, HRV, elevated heart rate, HR, at rest, sleeping HR above resting heart rate, RHR.

12. The wearable computing device of claim 8, wherein the at least one action includes at least one of generating an interface, providing a notification, modifying an operation of the wearable device, providing a recommendation for the user, or transmitting data for analysis.

## Patentansprüche

1. Verfahren zum Berechnen einer Stressbewertung für einen Benutzer einer tragbaren Vorrichtung, wobei das Verfahren Folgendes umfasst:
Empfangen (704) von ersten Merkmalsdaten, die einem Zustand des Benutzers der tragbaren Vorrichtung entsprechen, von einem oder mehreren externen Sensoren auf der tragbaren Vorrichtung, wobei die ersten Merkmalsdaten elektrodermale Aktivitätsdaten (electro-dermal activity, EDA), die unter Verwendung mindestens eines externen EDA-Sensors auf der tragbaren Vorrichtung erfasst werden, beinhalten;
Erlangen (706) von zweiten Merkmalsdaten, die dem Zustand des Benutzers entsprechen, über einen Prozessor der tragbaren Vorrichtung, wobei die zweiten Merkmalsdaten durch den Benutzer bereitgestellt werden und wobei die ersten Merkmalsdaten und die zweiten Merkmalsdaten Merkmalsdaten aus Schlafmerkmalen, Aktivitätsmerkmalen und Herzmerkmalen umfassen;
Berechnen (708) einer Stressbewertung unter Verwendung der ersten Merkmalsdaten und der zweiten Merkmalsdaten über den Prozessor der tragbaren Vorrichtung, wobei die Stressbewertung repräsentativ für einen Stresszustand des Benutzers ist und wobei das Berechnen (708) der Stressbewertung unter Verwendung der ersten Merkmalsdaten und der zweiten Merkmalsdaten ferner umfasst, dass die Stressbewertung als gewichtete Summe der Merkmalsdaten aus den Schlafmerkmalen, den Aktivitätsmerkmalen und den Herzmerkmalen berechnet wird;
und
Durchführen (710) mindestens einer Aktion basierend auf mindestens einem Teil des berechneten Stresswerts über den Prozessor der tragbaren Vorrichtung, **dadurch gekennzeichnet, dass** eine Gewichtung der Merkmalsdaten für verschiedene Benutzertypen bestimmt wird.

2. Verfahren nach Anspruch 1, wobei der mindestens eine externe EDA-Sensor an einer Seite der tragbaren Vorrichtung angebracht ist, die von einem Handgelenk des Benutzers entfernt ist.

3. Verfahren nach Anspruch 1, wobei die Schlafmerkmale mindestens eines von Unruhe, Fragmentierung, Schlafreservoirgrad, Tief-/REM-Schlafdauer, Tiefschlaflatenzzeit oder Vorkommen von Albträumen umfassen.

4. Verfahren nach Anspruch 1, wobei die Aktivitätsmerkmale mindestens eines von aktiven Zonenminuten oder Aktivitätsgrad, Trainings- oder Aktivitätsmetrik, Anstrengungsmetrik, Aktivitätstyp, Bewegungsmustern oder Schrittzählung oder Bewegung umfassen.

5. Verfahren nach Anspruch 1, wobei die Herzmerkmale mindestens eines von tiefer Schlaf-Herzfrequenz-Variabilität (heart rate variability, HRV), erhöhter Herzfrequenz (heart rate, HR), in Ruhe, Schlaf-HR über der Ruheherzfrequenz (resting heart rate, RHR) umfassen.

6. Verfahren nach einem der vorhergehenden Ansprüche, wobei die ersten Merkmalsdaten und die zweiten Merkmalsdaten Merkmalsdaten umfassen, die aus mindestens einem von Folgendem ausgewählt sind: Stoffwechsel-Daten, Blutzuckerspiegel, Körpergewicht oder -zusammensetzung, psychischer Zustand, wahrgenommenem Stress, Depression, Stimmprosodie/Ton/Druck, Cortisol-/Adrenalin-/Noradrenalin-Spiegel im Blut, Low-Density-Lipoprotein (low-density lipoprotein, LDL), BMI x Bewegung, geschlechtsspezifischen Werten oder Stimmungsprotokolldaten.

7. Verfahren nach einem der vorhergehenden Ansprüche, wobei die mindestens eine Aktion mindestens eines von Erzeugen einer Schnittstelle, Bereitstellen einer Benachrichtigung, Modifizieren eines Vorgangs der tragbaren Vorrichtung, Bereitstellen einer Empfehlung für den Benutzer oder Übertragen von Daten zur Analyse beinhaltet.

8. Tragbare Rechenvorrichtung, umfassend:
einen oder mehrere Sensoren;
mindestens einen Prozessor (902); und
mindestens eine Speichervorrichtung (904), die Anweisungen umfasst, die, wenn sie durch den mindestens einen Prozessor (902) ausgeführt werden, die tragbare Rechenvorrichtung zu Folgendem veranlassen:
Empfangen (704) von ersten Merkmalsdaten, die einem Zustand eines Benutzers der tragbaren Vorrichtung entsprechen, von dem einen oder den mehreren Sensoren, wobei die ersten Merkmalsdaten elektrodermale Aktivitätsdaten, EDA, die unter Verwendung eines EDA-Sensors auf der tragbaren Vorrichtung erfasst werden, beinhalten;
Erlangen (706) von zweiten Merkmalsdaten, die dem Zustand des Benutzers entsprechen, wobei die zweiten Merkmalsdaten durch den Benutzer bereitgestellt werden, wobei die ersten Merkmalsdaten und die zweiten Merkmalsdaten Merkmalsdaten aus Schlafmerkmalen, Aktivitätsmerkmalen und Herzmerkmalen umfassen;
Berechnen (708) einer Stressbewertung unter Verwendung der ersten Merkmalsdaten und der zweiten Merkmalsdaten, wobei die Stressbewertung repräsentativ für einen Stresszustand des Benutzers ist, wobei die Anweisungen ferner die tragbare Rechenvorrichtung veranlassen, die Stressbewertung als gewichtete Summe der Merkmalsdaten aus den Schlafmerkmalen, den Aktivitätsmerkmalen und den Herzmerkmalen zu berechnen;
und
Durchführen (710) mindestens einer Aktion basierend auf mindestens einem Teil des berechneten Stresswerts, **dadurch gekennzeichnet, dass** eine Gewichtung der Merkmalsdaten für verschiedene Benutzertypen bestimmt wird.

9. Tragbare Rechenvorrichtung nach Anspruch 8, wobei die Schlafmerkmale mindestens eines von Unruhe, Fragmentierung, Schlafreservoirgrad, Tief-/REM-Schlafdauer, Tiefschlaflatenzzeit oder Vorkommen von Albträumen umfassen.

10. Tragbare Rechenvorrichtung nach Anspruch 8, wobei die Aktivitätsmerkmale mindestens eines von aktiven Zonenminuten oder Aktivitätsgrad, Trainings- oder Aktivitätsmetrik, Anstrengungsmetrik, Aktivitätstyp, Bewegungsmustern oder Schrittzählung oder Bewegung umfassen.

11. Tragbare Rechenvorrichtung nach Anspruch 8, wobei die Herzmerkmale mindestens eines von tiefer Schlaf-Herzfrequenz-Variabilität (heart rate variability, HRV), erhöhter Herzfrequenz (heart rate, HR), in Ruhe, Schlaf-HR über der Ruheherzfrequenz (resting heart rate, RHR) umfassen.

12. Tragbare Rechenvorrichtung nach Anspruch 8, wobei die mindestens eine Aktion mindestens eines von Erzeugen einer Schnittstelle, Bereitstellen einer Benachrichtigung, Modifizieren eines Vorgangs der tragbaren Vorrichtung, Bereitstellen einer Empfehlung für den Benutzer oder Übertragen von Daten zur Analyse beinhaltet.

## Revendications

1. Procédé de calcul d'un score de stress pour un utilisateur d'un dispositif portable, le procédé comprenant :
la réception (704), à partir d'un ou plusieurs capteurs externes sur le dispositif portable, de premières données de caractéristiques correspondant à un état de l'utilisateur du dispositif portable, dans lequel les premières données de caractéristiques comportent des données d'activité électrodermique, EDA, capturées à l'aide d'au moins un capteur EDA externe sur le dispositif portable ;
l'obtention (706), via un processeur du dispositif portable, de secondes données de caractéristiques correspondant à l'état de l'utilisateur, les secondes données de caractéristiques fournies par l'utilisateur, et dans lequel les premières données de caractéristiques et les secondes données de caractéristiques comprennent des données de caractéristiques provenant de caractéristiques de sommeil, de caractéristiques d'activité et de caractéristiques cardiaques ;
le calcul (708), via le processeur du dispositif portable, d'un score de stress à l'aide des premières données de caractéristiques et des secondes données de caractéristiques, dans lequel le score de stress est représentatif d'un état de stress de l'utilisateur, et dans lequel le calcul (708) du score de stress à l'aide des premières données de caractéristiques et des secondes données de caractéristiques comprennent également le calcul du score de stress comme une somme pondérée des données de caractéristiques des caractéristiques de sommeil, des caractéristiques d'activité et des caractéristiques cardiaques ;
et
la réalisation (710), via le processeur du dispositif portable, d'au moins une action basée au moins en partie sur le score de stress calculé, **caractérisé en ce qu'**une pondération des données de caractéristiques est déterminée pour différents types d'utilisateurs.

2. Procédé selon la revendication 1, dans lequel l'au moins un capteur EDA externe est monté sur un côté du dispositif portable loin du poignet de l'utilisateur.

3. Procédé selon la revendication 1, dans lequel les caractéristiques du sommeil comprennent au moins l'un d'une agitation, d'une fragmentation, d'un niveau de réserve de sommeil, d'une durée du sommeil profond/paradoxal, d'une latence du sommeil profond ou d'une occurrence de cauchemar.

4. Procédé selon la revendication 1, dans lequel les caractéristiques d'activité comprennent au moins l'un de minutes de zone active ou d'un niveau d'activité, de mesures d'exercice ou d'activité, de mesures d'effort, d'un type d'activité, de modèles de mouvement ou d'un nombre de pas ou de mouvements.

5. Procédé selon la revendication 1, dans lequel les caractéristiques cardiaques comprennent au moins l'une d'une variabilité de fréquence cardiaque, HRV, en sommeil profond, d'une fréquence cardiaque, HR, élevée au repos, d'une HR en sommeil supérieure à la fréquence cardiaque au repos, RHR.

6. Procédé selon l'une quelconque des revendications précédentes, dans lequel les premières données et les secondes données comprennent des données sélectionnées parmi au moins l'un d'un score de fatigue physique, d'une pression artérielle, d'une composition sanguine, d'une fréquence respiratoire, d'une température, de données métaboliques, d'un taux de sucre dans le sang, d'un poids ou d'une composition corporelle, d'un état psychologique, d'un stress perçu, d'une dépression, d'une prosodie/d'un ton/d'une pression vocale, d'un taux de cortisol/d'épinéphrine/de norépinéphrine dans le sang, d'un taux de lipoprotéines de faible densité, LDL, d'un IMC croisé avec l'exercice physique, de valeurs spécifiques au sexe, ou de données de journal d'humeur.

7. Procédé selon l'une quelconque des revendications précédentes, dans lequel l'au moins une action comporte au moins l'une d'une génération d'une interface, d'une fourniture d'une notification, d'une modification d'une opération du dispositif portable, d'une fourniture d'une recommandation à l'utilisateur ou d'une transmission de données à des fins d'analyse.

8. Dispositif informatique portable, comprenant :
un ou plusieurs capteurs ;
au moins un processeur (902) ; et
au moins un dispositif de mémoire (904) stockant des instructions qui, lorsqu'elles sont exécutées par l'au moins un processeur (902), amènent le dispositif informatique portable à :
recevoir (704), à partir d'un ou de plusieurs capteurs, des premières données de caractéristiques correspondant à un état de l'utilisateur du dispositif portable, dans lequel les premières données de caractéristiques comportent des données d'activité électrodermique, EDA, capturées à l'aide d'un capteur EDA externe sur le dispositif portable ;
obtenir (706) des secondes données de caractéristiques correspondant à l'état de l'utilisateur, les secondes données de caractéristiques fournies par l'utilisateur, dans lequel les premières données de caractéristiques et les secondes données de caractéristiques comprennent des données de caractéristiques provenant de caractéristiques de sommeil, de caractéristiques d'activité et de caractéristiques cardiaques ;
calculer (708) un score de stress à l'aide des premières données de caractéristiques et des secondes données de caractéristiques, dans lequel le score de stress est représentatif d'un état de stress de l'utilisateur, dans lequel les instructions amènent également le dispositif informatique portable à calculer le score de stress comme une somme pondérée des données de caractéristiques des caractéristiques de sommeil, des caractéristiques d'activité et des caractéristiques cardiaques ;
et
réaliser (710) au moins une action basée au moins en partie sur le score de stress calculé, **caractérisé en ce qu'**une pondération des données de caractéristiques est déterminée pour différents types d'utilisateurs.

9. Dispositif informatique portable selon la revendication 8, dans lequel les caractéristiques du sommeil comprennent au moins l'un d'une agitation, d'une fragmentation, d'un niveau de réserve de sommeil, d'une durée du sommeil profond/paradoxal, d'une latence du sommeil profond ou d'une occurrence de cauchemar.

10. Dispositif informatique portable selon la revendication 8, dans lequel les caractéristiques d'activité comprennent au moins l'un de minutes de zone active ou d'un niveau d'activité, de mesures d'exercice ou d'activité, de mesures d'effort, d'un type d'activité, de modèles de mouvement ou d'un nombre de pas ou de mouvements.

11. Dispositif informatique portable selon la revendication 8, dans lequel les caractéristiques cardiaques comprennent au moins l'une d'une variabilité de fréquence cardiaque, HRV, en sommeil profond, d'une fréquence cardiaque, HR, élevée au repos, d'une HR en sommeil supérieure à la fréquence cardiaque au repos, RHR.

12. Dispositif informatique portable selon la revendication 8, dans lequel l'au moins une action comporte au moins l'une d'une génération d'une interface, d'une fourniture d'une notification, d'une modification d'une opération du dispositif portable, d'une fourniture d'une recommandation à l'utilisateur ou d'une transmission de données à des fins d'analyse.
